# EUROPÄISCHE PATENTSCHRIFT

(11) **EP 0 965 322 B1**
(45) Veröffentlichungstag und Bekanntmachung des Hinweises auf die Patenterteilung: **02.05.2003**
(21) Anmeldenummer: 99105722.5
(22) Anmeldetag: 20.03.1999
(51) Int. Cl.: A61K 7/06, A61K 7/48, A61K 7/50

(54) **Haar- und Körperreinigungsmittel mit verminderter Hautirritation**
Hair cleansing composition and personal cleansing composition with low skin irritancy
Shampooing ou composition nettoyante à faible irritation de la peau

(30) Priorität: 24.04.1998 DE 19818410
(43) Veröffentlichungstag der Anmeldung: 22.12.1999
(73) Patentinhaber: Wella Aktiengesellschaft, 64274 Darmstadt (DE)
(72) Erfinder: Irrgang, Bernhard, Dr., 1713 St. Anton (CH); Karlen, Thomas, Dr., 3013 Bern (CH); Pasquier, Gilbert, 1724 Praroman (CH)

(56) Entgegenhaltungen:
- US-A- 4 948 576
- US-A- 5 391 368
- US-A- 5 641 479
- US-A- 5 656 257

## Beschreibung

Gegenstand der Erfindung ist ein Haar- und Körperreinigungsmittel, das ein spezielles Homo- oder Copolymeres in Kombination mit einem Tensidgemisch enthält.

Kosmetische Zubereitungen, vor allem solche, die zur Haar- und Körperreinigung verwendet werden, wie z.B. Dusch- und Schaumbäder, Haarshampoos und andere Haarpflegemittel enthalten als Basis meist anionische Tenside wie Alkylsulfate, alpha-Olefinsulfonate und Alkylethersulfate. Die Anforderung an solche Mittel liegt vor allem darin, auf der Haut und in den Haaren befindliche Schweiß-, Fett- und Schmutzpartikel zu entfernen, ohne dabei Hautirritationen hervorzurufen. Es ist aber von Zubereitungen, welche die oben genannten anionischen Tenside enthalten, bekannt, daß sie in gewissem Umfange zu Hautirritationen führen können. Deshalb hat man in den letzten Jahren verstärkt hautfreundlichere Formulierungen für den täglichen Gebrauch entwickelt, in denen sogenannte Sekundärtenside die oben genannten anionischen Tenside teilweise ersetzen. Die Stoffe sind in ihrer Irritationswirkung wesentlich geringer einzuschätzen. Zu nennen sind z.B. Alkylpolyglucoside, Betaine, Glycinate, Proteinderivate, Ethercarboxylate und Sulfosuccinate. Sollen hingegen nahezu irritationsfreie Formulierungen entwickelt werden, so muß man ganz auf Alkylsulfate, Alkylethersulfate bzw. alpha-Olefinsulfonate verzichten. Bei alleiniger Verwendung der oben genannten Sekundärtenside sinkt zwar die Irritationswirkung erheblich, jedoch sind anwendungstechische Nachteile wie ein schlechtes Schaumvermögen in Kauf zu nehmen. Von der Seite der Konsumenten wird aber bei Shampoos und Duschbädern ein gutes Schaumvermögen gefordert. Eine Zubereitung mit schlechtem Schaumvermögen wird oft als qualitativ minderwertig eingestuft. Es sind zwar Substanzen bekannt, die das Schaumvermögen einer schwach schäumenden Tensidzusammensetzung steigern können und die als "foam booster" bezeichnet werden. Hierzu zählen zum Beispiel die Fettsäurealkanolamide und Aminoxide. Wegen ihres unvorteilhaften toxikologischen Profils ist man jedoch bestrebt, einen Einsatz der Alkanolamide und Aminoxide in zeitgemäßen Haar- und Körperreinigungsmitteln zu vermeiden.

Es bestand daher die Aufgabe, Haar- und Körperreinigungsmittel zur Verfügung zu stellen, welche eine möglichst geringe hautirritierende Wirkung aufweisen und gleichzeitig keine oder möglichst geringe Einbußen in ihren anderen, gewünschten Eigenschaften (Reinigungsvermögen, Schäumvermögen, Viskositätsverhalten, toxikologische Unbedenklichkeit etc.) zeigen.

Es wurde gefunden, daß die Aufgabe gelöst wird durch ein Haar- und Körperreinigungsmittel mit einem Gehalt an
(A) einem Homo- oder Copolymeren, gebildet aus mindestens einem ethylenisch ungesättigten Monomeren der allgemeinen Formel (I)

   CH₂=CR¹R² (I)

   wobei R¹ ausgewählt ist aus A-(CH₂CH₂O)ₓ-R³ und COOH, A ausgewählt ist aus C(=O)O, C(=O)NH und CH₂O, x eine Zahl von 1 bis 100, vorzugsweise von 10 bis 50 ist, R³ einen C1- bis C30-Alkylrest bedeutet, R² ausgewählt ist aus H, C1-C30-Alkyl und CH₂-R¹, unter der Maßgabe, daß mindestens einer der Reste R¹ und R² die Gruppe A-(CH₂CH₂O)ₓ-R³ enthält,
(B) mindestens einem nichtionischen Tensid der allgemeinen Formel (II)

   R⁴-O-(CHR⁵-CH₂-O)ₙ-H (II)

   wobei R4 eine gesättigte oder ungesättigte, lineare oder verzweigte Alkylgruppe mit 6 bis 20 Kohlenstoffatomen bedeutet, die mit Hydroxylgruppen substituiert sein kann, R5 Wasserstoff oder Methyl bedeutet und n eine Zahl von 1 bis 10, vorzugsweise von 1 bis 6 ist und
(C) mindestens einem von (B) verschiedenen Tensid oder einer Mischung von Tensiden mit reinigender Wirkung und keiner oder sehr geringer hautirritierender Wirkung.

Komponente (A) ist vorzugsweise in einer Menge von 0,01 bis 10 Gewichtsprozent, besonders bevorzugt in einer Menge von 0,05 bis 5 Gewichtsprozent enthalten.

Vorzugsweise ist Komponente (A) ein Copolymer, welches gebildet ist aus mindestens einem ethylenisch ungesättigten Monomeren der allgemeinen Formel (I) sowie mindestens einem ethylenisch ungesättigten Monomeren der allgemeinen Formel (III)

CH₂=C(R⁶)COOR⁷ (III)

wobei R6 und R7 unabhängig voneinander ausgewählt sind aus H und einer Alkylgruppe mit 1 bis 30, vorzugsweise mit 1 bis 12, insbesondere bevorzugt mit 1 bis 4 Kohlenstoffatomen.

Bevorzugt ist weiterhin, daß die Gruppe A in Formel (I) ausgewählt ist aus C(=O)O und CH₂O, daß R² ausgewählt ist aus H und Methyl, daß R³ einen C8- bis C30-Alkylrest bedeutet oder daß es sich bei dem Monomer der Formel (I) um ein Itaconsäurederivat handelt. Ebenfalls bevorzugt ist, daß es sich bei dem Monomer der Formel (III) um Acrylsäure, Methacrylsäure oder einen ihrer C1- bis C4-Alkylester handelt.

Geeignete Copolymere sind beispielsweise Acryl- oder Methacrylsäure/Acryl- oder Methacrylsäurepolyethoxyalkylester Copolymere (INCI-Bezeichnung: Acrylates/Steareth-20 Methacrylate Copolymer), wie sie von der Firma Rohm und Haas/USA unter den Bezeichungen Acrysol®-22, Acrysol® ICS oder Aculyn®-22 vertrieben werden oder Acryl- oder Methacrylsäure/Polyethoxyalkylallylether Copolymere (INCI-Bezeichnung: Steareth-10 Allyl Ether/Acrylates Copolymer), wie sie von der Firma Allied Colloids/Großbritannien unter der Bezeichnung Salcare® SC 90 vertrieben werden oder Acryl- oder Methacrylsäure/Itaconsäurepolyethoxyalkylester Copolymere (INCI-Bezeichnungen: Acrylates/Steareth-20 Itaconate Copolymer und Acrylates/Ceteth-20 Itaconate Copolymer), wie sie von der Firma National Starch/USA unter den Bezeichnungen Structure® 2001 und Structure® 3001 vertrieben werden.

Die Acryl- oder Methacrylsäuregruppen sind in den eingesetzten Polymeren vorzugsweise durch organische oder anorganische Basen, insbesondere durch primäre oder sekundäre Amine, z.B. durch Monoethanolamin, Aminomethylpropanol (AMP), Triethanolamin oder auch durch wäßrige Lösungen von Erdalkalihydroxiden oder Ammoniak neutralisiert.

Komponente (B) ist vorzugsweise in einer Menge von 0,01 bis 20 Gewichtsprozent, besonders bevorzugt in einer Menge von 0,05 bis 10 Gewichtsprozent enthalten.

Bei Komponente (B) handelt es sich vorzugsweise um alkoxylierte, insbesondere ethoxylierte Fettalkohole, wobei die Fettalkylgruppe 6 bis 20 Kohlenstoffatome enthält und der Alkoxylierungs- bzw. Ethoxylierungsgrad 1 bis 10, vorzugsweise 1 bis 6 beträgt. Beispiele für geeignete Fettalkoholethoxylate sind oxethylierter Lauryl-, Tetradecyl-, Cetyl-, Oleyl- oder Stearylalkohol, die allein oder im Gemisch eingesetzt werden können. Auch die ethoxylierten Fettalkohole, die unter der Typenbezeichnung Dehydol® von der Firma Henkel oder unter der Typenbezeichnung Brij® von der Firma ICI Surfactants vertrieben werden, sind für das erfindungsgemäße Haarreinigungsmittel geeignet. Besonders bevorzugt sind Polyethylenglykol (3) laurylether (INCI-Name: Laureth-3) und Polyethylenglykol(4)laurylether (INCI-Name: Laureth-4).

Die Tenside der Komponente (C) können einzeln oder in Kombination vorliegen und sind in einer Menge von vorzugsweise 1 bis 50 Gewichtsprozent, besonders bevorzugt von 1 bis 30 Gewichtsprozent enthalten. Als Tensid gemäß Komponente (C) sind insbesondere nichtionische, amphotere, zwitterionische und anionische Tenside geeignet.

Komponente (C) ist vorzugsweise frei von waschaktiven Tensiden mit hautirritierender Wirkung wie Alkylsulfaten, Alkylethersulfaten und alpha-Olefinsulfonaten oder enthält derartige Tenside nur in geringen, keine Hautirritation hervorrufenden Mengen von bis zu 1 bis 3 Gewichtsprozent.

Geeignete nicht-ionische Tenside sind z.B. alkoxylierte Fettalkohole mit einem hohen Alkoxylierungsgrad, z.B. von 11 bis 50 sowie alkoxylierte Fettsäureester, alkoxylierte Partialglyceride von verzweigten oder unverzweigten, gesättigten oder ungesättigten C6- bis C20-Fettsäuren und einem Alkoxierungsgrad von 11 bis 400 wie z.B. Polyethylenglykol(200)glycerylpalmitat, alkoxylierte Polyolester wie z.B. ethoxylierte Zuckerester, beispielsweise Polyethylenglykol(120)-methylglucosedioleat und Alkylpolyglucoside wie z.B. Coco-Glucoside, Lauryl-Glucoside oder Decyl-Glucoside. Beispiele für geeignete Fettalkoholethoxylate sind oxethylierter Lauryl-, Tetradecyl-, Cetyl-, Oleyl- oder Stearylalkohol, die allein oder im Gemisch eingesetzt werden können, sowie Fettalkohole von oxethyliertem Lanolin oder oxethyliertes Lanolin. Auch die ethoxylierten Fettalkohole, die unter der Typenbezeichnung Dehydol® von der Firma Henkel oder unter der Typenbezeichnung Brij® von der Firma ICI Surfactants vertrieben werden, sind für das erfindungsgemäße Haarbehandlungsmittel geeignet, sofern sie einen genügend hohen Ethoxylierungsgrad aufweisen.

Unter den Fettsäureesterethoxylaten sind vor allem Diglyceridethoxylate zu nennen wie das von der ICI Surfactants unter dem Handelnamen Arlatone® G vertriebene, mit 25 Ethylenoxid-Einheiten ethoxylierte Rizinusöl mit dem INCI-Namen PEG-25 Hydrogenated Castor Oil, das von der BASF unter dem Handelnamen Cremophor® EL vertriebene, mit 35 Ethylenoxid-Einheiten ethoxylierte Rizinusöl mit dem INCI-Namen PEG-35 Castor Oil, das von der BASF unter dem Handelsnamen Cremophor® RH 410 vertriebene, mit 40 Ethylenoxid-Einheiten ethoxylierte, hydrierte Rizinusöl mit dem INCI-Namen PEG-40 Hydrogenated Castor Oil, und die von der Firma Witco Surfactants unter dem Namen Rewoderm® LI vertriebenen Rohstoffe zu nennen.

Weiterhin können die als nicht-ionische Tenside bekannten ethoxylierten Fettsäurezuckerester, insbesondere der ethoxylierte Sorbitanfettsäureester, aber auch nicht ethoxylierte Tenside, wie die Fettsäurezuckerester, die von der Firma ICI Surfactants unter dem Handelsnamen Tween® und Arlacel® vertrieben werden sowie die Alkylpolyglycoside, die von der Firma Henkel unter dem Handelsnamen Plantaren® oder Plantacare® oder von der Firma Seppic unter dem Handelsnamen Oramix® vertrieben werden, für die erfindungsgemäße kosmetische Zubereitung eingesetzt werden.

Geeignete amphotere Tenside sind beispielsweise Betaine wie Cocamidopropylbetain oder Laurylbetain, Sulfobetaine wie z.B. Cocamidopropyl Hydroxysultaine, Glycinate wie z.B. Cocoamphoglycinat (INCI-Bezeichnung: Sodium Cocoamphoacetate) und -diglycinat sowie Propionate wie z.B. Cocoamphopropionat.

Geeignete anionische Tenside sind z.B. Sulfosuccinate wie Disodium Laureth-3 Sulfosuccinat, Disodium PEG-5 Laurylcitrat Sulfosuccinat, Disodium Ricinolamido MEA-Sulfosuccinat oder Disodium Laurylamido MEA-Sulfosuccinat und Ethercarboxylate wie z.B. Sodium Laureth-6 Carboxylat oder Sodium Laureth-11 Carboxylat.

Desweiteren können Zusatz- und Hilfsstoffe, wie sie in kosmetischen Formulierungen üblich sind, verwendet werden. Zu nennen sind Pflegestoffe wie quaternisierte Alkylamine, kationische Polymere natürlichen oder synthetischen Ursprungs, Proteine und ihre Derivate, wie z.B. Kollagen-, Keratin-, Seidenprotein- und Weizenproteinhydrolysate. Desweiteren können eingesetzt werden: Parfümöle, Farbstoffe, Trübungsmittel wie z.B. Glycoldistearat; weitere Verdicker wie Guar Gums, Silikate, Methylcellulose, Hydroxyethylcellulose, Carboxyvinylpolymere; weitere haarkonditionierende Mittel wie synthetische oder natürliche Phospholipide oder quaternäre Derivate der Stärke oder Cellulose; Lösungsvermittler wie kurzkettige Alkohole, z.B. Ethanol, n- und iso-Propanol oder Glykole wie Butylenoder Propylenglykol; Aminosäuren wie z.B. Histidin, Glycin, Alanin, Threonin, Arginin, Cystein und deren Derivate wie z.B. Fettsäurekondensationsprodukte oder quaternäre Produkte; weitere Wirkstoffe wie Pflanzenextrakte, Vitamine, Allantoin, Chitosan, Konservierungsmittel u.v.m..

Der Wassergehalt des erfindungsgemäßen Haar- und Körperreinigungsmittels liegt vorzugsweise zwischen 15 und 80 Gewichtsprozent.

Das erfindungsgemäße Mittel weist vorzugsweise einen pH-Wert von 4 bis 9, besonders bevorzugt von 4,5 bis 7,5 auf. Die Einstellung des pH-Wertes des erfindungsgemäßen Mittels kann beispielsweise mit Zitronensäure, Phosphorsäure, Salzsäure oder Natriumhydroxid erfolgen.

Der Zusatz der Komponenten (A) und (B) bewirkt eine signifikante Erhöhung der Schaummenge einer an sich schwach schäumenden Tensidmischung. Daher ist ein weiterer Gegenstand der Erfindung die Verwendung einer Kombination der oben definierten Komponenten (A) und (B) zur Verbesserung der Schäumeigenschaften von Tensidzusammensetzungen.

Das erfindungsgemäße Haar- und Körperreinigungsmittel zeichnet sich insbesondere durch eine sehr gute Hautverträglichkeit aus. In einem Duhring-Kammer-Test wurden gegenüber einer Laurylethersulfat enthaltenden Vergleichsrezeptur wesentlich bessere Resultate erzielt. Die Hautverträglichkeit war derjenigen von Wasser vergleichbar. Daher ist ein weiterer Gegenstand der Erfindung die Verwendung einer Kombination der oben definierten Komponenten (A) und (B) zur Herstellung von Haar- und Körperreinigungsmitteln mit vermindertem Hautirritationspotential.

Die nachfolgenden Beispiele sollen den Gegenstand der Erfindung näher erläutern.

### Beispiele

Die Bestimmung der Schaummenge erfolgte in Anlehnung an die Ross-Miles-Methode (nach DIN 53902, Teil 2). Die verwendete Konzentration betrug ca. 0,1 Gew.% (aktiv).

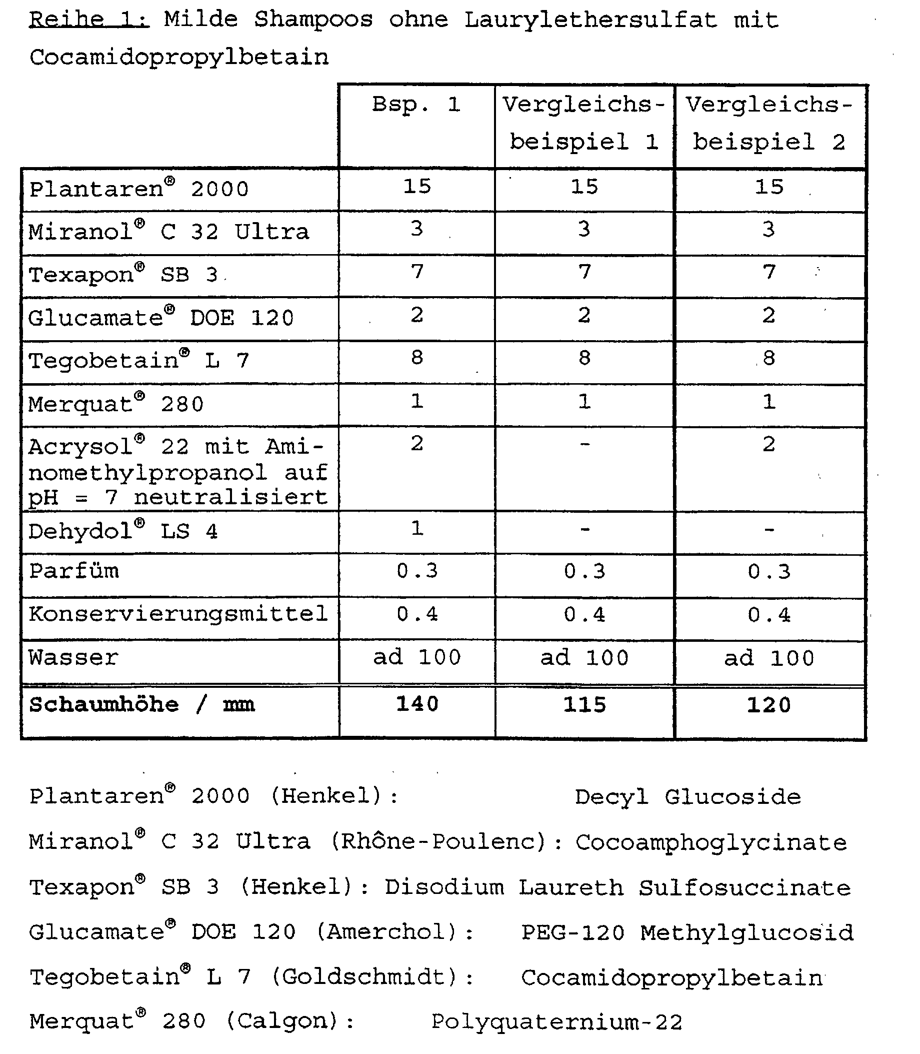

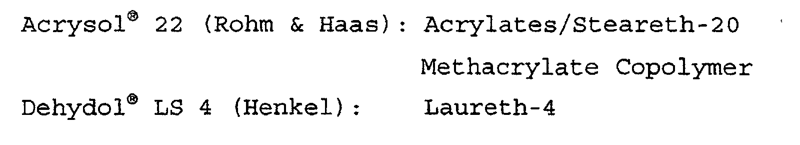

Aus obiger Tabelle ist zu entnehmen, daß eine Zugabe von Acrylsol® 22 und Dehydol® LS 4 zum Vergleichsbeispiel 1 die Schaummenge signifikant erhöht (um ca. 20 %). Acrysol® 22 allein (Vergleichsbeispiel 2) führt dagegen nur zu einer nicht-signifikanten Steigerung der Schaummenge gegenüber Vergleichsbeispiel 1.

Diese Ergebnisse wurden auch im Halbseitentest unter Verwendung verschiedener Haarqualitäten bestätigt. Ein Verzicht auf Dehydol® LS 4 und/oder Acrysol® 22 führt auf dem Kopf zu einer Verminderung der Schaummenge.

Mit der Basisrezeptur wurde ein Duhring-Kammer-Test durchgeführt. Diese Rezeptur schnitt wesentlich besser ab als eine Vergleichsrezeptur, die als Basistensid Laurylethersulfat verwendete. Die Hautverträglichkeit war derjenigen von Wasser vergleichbar.

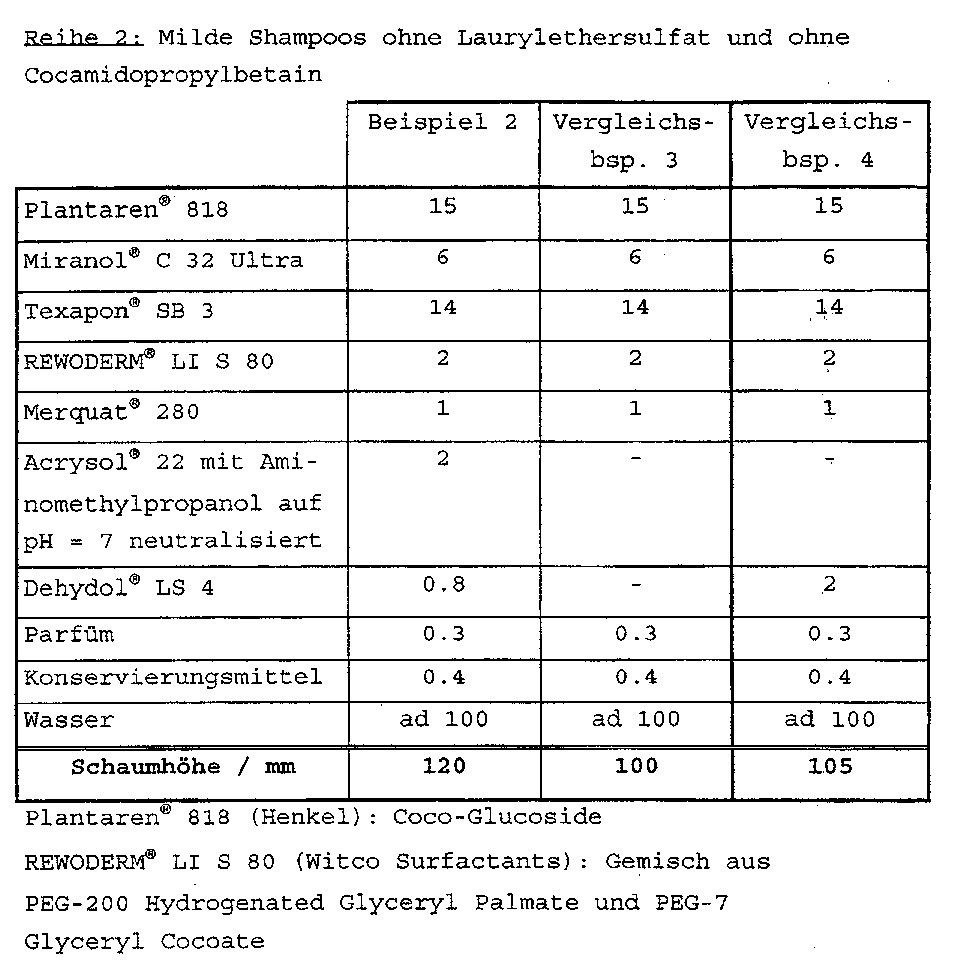

Auch hier ist bei der Verwendung von Acrysol 22 und Dehydol LS 4 eine Steigerung der Schaummenge um über 20 % festzustellen, die bei Verwendung von Dehydol LS 4 allein nicht erreicht wird.

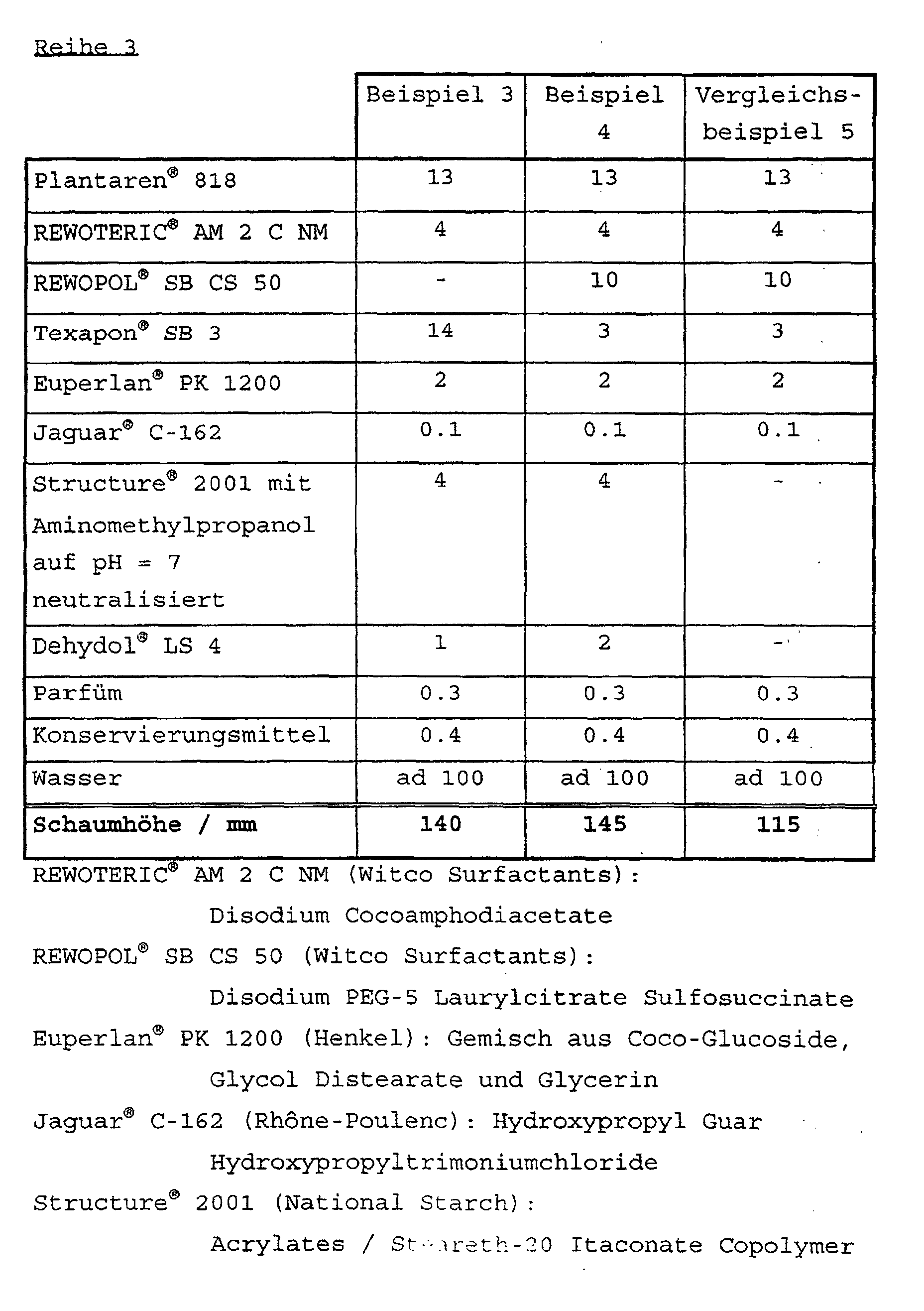

## Patentansprüche

1. Haar- und Körperreinigungsmittel mit einem Gehalt an
(A) einem Homo- oder Copolymeren, gebildet aus mindestens einem ethylenisch ungesättigten Monomeren der allgemeinen Formel (I)
CH₂=CR¹R² (I)
wobei R¹ ausgewählt ist aus A-(CH₂CH₂O)ₓ-R³ und COOH, A ausgewählt ist aus C(=O)O, C(=O)NH und CH₂O, x eine Zahl von 1 bis 100 ist, R³ einen C1-bis C30-Alkylrest bedeutet, R² ausgewählt ist aus H, C1-C30-Alkyl und CH₂-R¹, unter der Maßgabe, daß mindestens einer der Reste R¹ und R² die Gruppe A-(CH₂CH₂O)ₓ-R³ enthält,
(B) mindestens einem nichtionischen Tensid der allgemeinen Formel (II)
R⁴-O-(CHR⁵-CH₂-O)ₙ-H (II)
wobei R4 eine gesättigte oder ungesättigte, lineare oder verzweigte Alkylgruppe mit 6 bis 20 Kohlenstoffatomen bedeutet, die mit Hydroxylgruppen substituiert sein kann, R5 Wasserstoff oder Methyl bedeutet und n eine Zahl von 1 bis 10 ist und
(C) mindestens einem von (B) verschiedenen Tensid oder einer Mischung von Tensiden mit reinigender Wirkung und keiner oder sehr geringer hautirritierender Wirkung.

2. Mittel nach Anspruch 1, **dadurch gekennzeichnet, daß** Komponente (A) ein Copolymer ist, gebildet aus mindestens einem ethylenisch ungesättigten Monomeren der allgemeinen Formel (I) sowie mindestens einem ethylenisch ungesättigten Monomeren der allgemeinen Formel (III)
CH₂=C(R⁶)COOR⁷ (III)
wobei R6 und R7 unabhängig voneinander ausgewählt sind aus H und C1- bis C30-Alkyl.

3. Mittel nach einem der vorstehenden Ansprüche, **dadurch gekennzeichnet, daß** A ausgewählt ist aus C(=O)O und CH₂O, R² ausgewählt ist aus H und Methyl und R³ einen C8- bis C30-Alkylrest bedeutet.

4. Mittel nach Anspruch 1 oder 2, **dadurch gekennzeichnet, daß** es sich bei dem Monomer der Formel (I) um ein Itaconsäurederivat handelt.

5. Mittel nach einem der vorstehenden Ansprüche, **dadurch gekennzeichnet, daß** das Monomer der Formel (III) Acrylsäure, Methacrylsäure oder einer deren C1- bis C4-Alkylester ist.

6. Mittel nach einem der vorstehenden Ansprüche, **dadurch gekennzeichnet, daß** Komponente (A) ausgewählt ist aus Acryl- oder Methacrylsäure/Acryl- oder Methacrylsäurepolyethoxyalkylester Copolymeren, Acryl- oder Methacrylsäure/Polyethoxyalkylallylether Copolymeren und Acryl- oder Methacrylsäure/Itaconsäurepolyethoxyalkylester Copolymeren.

7. Mittel nach einem der vorstehenden Ansprüche, **dadurch gekennzeichnet, daß** freie Carbonsäuregruppen der Komponente (A) in neutralisierter Form vorliegen.

8. Mittel nach einem der vorstehenden Ansprüche, **dadurch gekennzeichnet, daß** es Komponente (A) in einer Menge von 0,01 bis 10 Gewichtsprozent enthält.

9. Mittel nach einem der vorstehenden Ansprüche, **dadurch gekennzeichnet, daß** es Komponente (B) in einer Menge von 0,01 bis 20 Gewichtsprozent enthält.

10. Mittel nach einem der vorstehenden Ansprüche, **dadurch gekennzeichnet, daß** der Wert n in Formel (II) eine Zahl von 1 bis 6 bedeutet.

11. Mittel nach einem der vorstehenden Ansprüche, **dadurch gekennzeichnet, daß** es Komponente (C) in einer Menge von 1 bis 50 Gewichtsprozent enthält.

12. Mittel nach einem der vorstehenden Ansprüche, **dadurch gekennzeichnet, daß** es sich bei Komponente (C) um waschaktive nichtionische, amphotere, zwitterionische oder anionische Tenside handelt.

13. Mittel nach einem der vorstehenden Ansprüche, **dadurch gekennzeichnet, daß** es frei ist von waschaktiven Tensiden mit hautirritierender Wirkung wie Alkylsulfaten, Alkylethersulfaten und alpha-Olefinsulfonaten oder derartige Tenside nur in geringen, keine Hautirritation hervorrufenden Mengen enthält.

14. Verwendung einer Kombination der Komponenten (A) und (B) gemäß Anspruch 1 zur Verbesserung der Schäumeigenschaften von Tensidzusammensetzungen.

15. Verwendung einer Kombination der Komponenten (A) und (B) gemäß Anspruch 1 zur Herstellung von Haar-Körperreinigungsmitteln mit vermindertem Hautirritationspotential.

## Claims

1. Hair- and body-cleansing composition with a content of
(A) a homo- or copolymer formed from at least one ethylenically unsaturated monomer of the general formula (I)
CH₂=CR¹R² (I)
where R¹ is chosen from A-(CH₂CH₂O)ₓ-R³ and COOH, A is chosen from the groups C(=O)O, C(=O)NH and CH₂O, x is a number from 1 to 100, R³ is a C₁- to C₃₀-alkyl radical, R² is chosen from H, C₁-C₃₀-alkyl and CH₂-R¹, with the proviso that at least one of the radicals R¹ and R² contains the group A-(CH₂CH₂O)ₓR³,
(B) at least one nonionic surfactant of the general formula (II)
R⁴-O-(CHR⁵-CH₂-O)ₙ-H (II)
where R⁴ is a saturated or unsaturated, linear or branched alkyl group having 6 to 20 carbon atoms which may be substituted by hydroxyl groups, R⁵ is hydrogen or methyl and n is a number from 1 to 10, and
(C) at least one surfactant, or a mixture of surfactants, different from (B) having a cleansing effect and no or very slight skin-irritating effect.

2. Composition according to Claim 1, **characterized in that** component (A) is a copolymer formed from at least one ethylenically unsaturated monomer of the general formula (I), and at least one ethylenically unsaturated monomer of the general formula (III)
CH₂=C(R⁶)COOR⁷ (III)
where R⁶ and R⁷, independently of one another, are chosen from H and C₁- to C₃₀-alkyl.

3. Composition according to one of the preceding claims, **characterized in that** A is chosen from C(=O)O and CH₂O, R² is chosen from H and methyl and R³ is a C₈- to C₃₀-alkyl radical.

4. Composition acc ording to Claim 1 or 2, **characterized in that** the monomer of the formula (I) is an itaconic acid derivative.

5. Composition according to one of the preceding claims, **characterized in that** the monomer of the formula (III) is acrylic acid, methacrylic acid or one of their C₁- to C₄-alkyl esters.

6. Composition according to one of the preceding claims, **characterized in that** component (A) is chosen from acrylic acid or methacrylic acid/acrylic or methacrylic polyethoxyalkyl ester copolymers, acrylic acid or methacrylic acid/polyethoxyalkyl allyl ether copolymers and acrylic acid or methacrylic acid/itaconic polyethoxyalkyl ester copolymers.

7. Composition according to one of the preceding claims, **characterized in that** free carboxylic acid groups of component (A) are in neutralized form.

8. Composition according to one of the preceding claims, **characterized in that** it comprises component (A) in an amount of from 0.01 to 10% by weight.

9. Composition according to one of the preceding claims, **characterized in that** it comprises component (B) in an amount of from 0.01 to 20% by weight.

10. Composition according to one of the preceding claims, **characterized in that** the value n in formula (II) is a number from 1 to 6.

11. Composition according to one of the preceding claims, **characterized in that** it comprises component (C) in an amount of from 1 to 50% by weight.

12. Composition according to one of the preceding claims, **characterized in that** component (C) is a washing-active nonionic, amphoteric, zwitterionic or anionic surfactant.

13. Composition according to one of the preceding claims, **characterized in that** it is free from washing-active surfactants having a skin-irritating effect, such as alkyl sulphates, alkyl ether sulphates and alpha-olefinsulphonates, or comprises such surfactants only in small amounts which do not cause skin irritation.

14. Use of a combination of components (A) and (B) according to Claim 1 for improving the foaming properties of surfactant compositions.

15. Use of a combination of components (A) and (B) according to Claim 1 for the preparation of hair- and body-cleansing compositions with reduced skin irritation potential.

## Revendications

1. Composition pour le nettoyage du corps et des cheveux, contenant
(A) un homo- ou copolymère constitué d'au moins un monomère à insaturation éthylénique de formule générale (I)
CH₂=CR¹R² (I)
dans laquelle R¹ est choisi parmi A-(CH₂CH₂O)ₓ-R³ et COOH, A est choisi parmi C(=O)O, C(=O)NH et CH₂O, x est un nombre allant de 1 à 100, R³ représente un radical alkyle en C₁-C₃₀, R² est choisi parmi H, un groupe alkyle en C₁-C₃₀ et CH₂-R¹, étant entendu qu'au moins un des radicaux R¹ et R² contient le groupe A-(CH₂CH₂O)ₓ-R³,
(B) au moins un tensioactif non ionique de formule générale (II)
R⁴-O-(CHR⁵-CH₂-O)ₙ-H (II)
dans laquelle R⁴ représente un groupe alkyle linéaire ou ramifié, saturé ou insaturé ayant de 6 à 20 atomes de carbone, qui peut être substitué par des groupes hydroxy, R⁵ représente un atome d'hydrogène ou le groupe méthyle et n est un nombre allant de 1 à 10 et
(C) au moins un tensioactif différent de (B) ou un mélange de tensioactifs ayant une action de nettoyage et aucune action ou une très faible action irritante sur la peau.

2. Composition selon la revendication 1, **caractérisée en ce que** le composant (A) est un copolymère constitué d'au moins un monomère à insaturation éthylénique de formule générale (I) ainsi que d'au moins un monomère à insaturation éthylénique de formule générale (III)
CH₂=C(R⁶)COOR⁷ (III)
dans laquelle R⁶ et R⁷ sont choisis, indépendamment l'un de l'autre, parmi H et un groupe alkyle en C₁-C₃₀.

3. Composition selon l'une quelconque des revendications précédentes, **caractérisée en ce que** A est choisi parmi C(=O)O et CH₂O, R² est choisi parmi H et le groupe méthyle et R³ représente un groupe alkyle en C₈-C₃₀.

4. Composition selon la revendication 1 ou 2, **caractérisée en ce que** le monomère de formule (I) consiste en un dérivé d'acide itaconique.

5. Composition selon l'une quelconque des revendications précédentes, **caractérisée en ce que** le monomère de formule (III) est l'acide acrylique, l'acide méthacrylique ou un de leurs esters d'alkyle en C₁-C₄.

6. Composition selon l'une quelconque des revendications précédentes, **caractérisée en ce que** le composant (A) est choisi parmi des copolymères acide acrylique ou méthacrylique/acrylate ou méthacrylate de polyéthoxyalkyle, des copolymères acide acrylique ou méthacrylique/polyéthoxyalkylallyléther et des copolymères acide acrylique ou méthacrylique/itaconate de polyéthoxyalkyle.

7. Composition selon l'une quelconque des revendications précédentes, **caractérisée en ce que** des groupes carboxy libres du composant (A) se trouvent sous forme neutralisée.

8. Composition selon l'une quelconque des revendications précédentes, **caractérisée en ce qu'**elle contient le composant (A) en une quantité de 0,1 à 10 % en poids.

9. Composition selon l'une quelconque des revendications précédentes, **caractérisée en ce qu'**elle contient le composant (B) en une quantité de 0,01 à 20 % en poids.

10. Composition selon l'une quelconque des revendications précédentes, **caractérisée en ce que** la valeur n dans la formule (II) représente un nombre de 1 à 6.

11. Composition selon l'une quelconque des revendications précédentes, **caractérisée en ce qu'**elle contient le composant (C) en une quantité de 1 à 50 % en poids.

12. Composition selon l'une quelconque des revendications précédentes, **caractérisée en ce que** le composant (C) consiste en des tensioactifs lavants non ioniques, amphotères, zwitterioniques ou anioniques.

13. Composition selon l'une quelconque des revendications précédentes, **caractérisée en ce qu'**elle est exempte de tensioactifs lavants à action irritante sur la peau, tels que des alkylsulfates, alkyléthersulfates et α-oléfinesulfonates ou ne contient de tels tensioactifs qu'en faibles quantités ne provoquant aucune irritation de la peau.

14. Utilisation d'une association des composants (A) et (B) selon la revendication 1, pour l'amélioration des propriétés de moussage de compositions de tensioactifs.

15. Utilisation d'une association des composants (A) et (B) selon la revendication 1, pour la préparation de compositions de nettoyage pour le corps et les cheveux, à potentiel réduit d'irritation de la peau.
